(19) Europäisches Patentamt
European Patent Office
Office européen des brevets

(11) Numéro de publication: 0 208 172
B1

(12) # FASCICULE DE BREVET EUROPÉEN

(45) Date de publication du fascicule du brevet: 28.02.90

(51) Int. Cl.[5]: A 61 L 25/00

(21) Numéro de dépôt: 86108193.3

(22) Date de dépôt: 16.06.86

(54) **Ciment pour fixation de prothèses d'os.**

(30) Priorité: 20.06.85 FR 8509397

(43) Date de publication de la demande: 14.01.87 Bulletin 87/03

(45) Mention de la délivrance du brevet: 28.02.90 Bulletin 90/09

(84) Etats contractants désignés: AT BE CH DE FR GB IT LI LU NL SE

(56) Documents cité:
EP-A-0 123 323
US-A-3 200 142
US-A-4 284 551
US-A-4 404 327

PATENTS ABSTRACTS OF JAPAN, vol. 5, no. 124 (C-66) 796 , 11 août 1981, page 146 C 66; & JP-A-56 61 470 (LION K.K.) 26-05-1981

CHEMICAL ABSTRACTS, vol. 98, no. 12, 21 mars 1983, page 373, résumé no. 95619u, Columbus, Ohio, US; S. KITOH et al.: "Studies of dental adhesive monomers. 2. Adhesiveness of 2-hydroxy-3-(N-substituted phenylamino)propyl methacrylates to tooth enamel", & KOBUNSHI RONBUNSHU 1982, 39(12), 817-19

(73) Titulaire: CERAVER Société anonyme dite:
10, quai Paul-Doumer
F-92400 Courbevoie (FR)

(72) Inventeur: Fontanille, Michel
71, rue de Ségur
F-33000 Bordeaux (FR)
Inventeur: Timar, Myrna
12, rue Fontaine
F-93200 Saint Denis (FR)

(74) Mandataire: Weinmiller, Jürgen
Lennéstrasse 9 Postfach 24
D-8133 Feldafing (DE)

EP 0 208 172 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dipposition (Art. 99(1) Convention sur le brevet européen).

LIBERGRAF, STOCKHOLM 1990

**Description**

La présente invention concerne un ciment pour fixation de prothèses d'os, notamment d'endoprothèses d'articulations, contenant:

a) une phase solide comprenant d'une part des poudres de polyméthacrylate de méthyle et d'un copolymère de méthacrylate de méthyle et de méthacrylate ou d'acrylate d'un autre alcool, et d'autre part un peroxyde organique générateur de radicaux libres, et
b) une phase liquide comprenant du méthacrylate de méthyle et un accélérateur de décomposition du peroxyde organique.

On a déjà proposé des ciments de ce genre dans le document GB-A-1 130 653. De tels ciments présentent cependant une réaction de polymérisation fortement exothermique, de sorte que leur température s'élève à environ 90°C au cours de leur prise après mise en place dans l'os. Une telle élévation de température entraîne une altération du tissu osseux environnant, qui exerce un effet néfaste pour la stabilité de la prothèse mise en place. Par ailleurs, ils comportent comme accélérateur de la décomposition du peroxyde organique une amine tertiaire ou un acide sulfinique aromatique, qui présentent une toxicité notable a l'égard de l'organisme.

Le brevet US-A-4 284 551 décrit par ailleurs un ciment pour applications dentaires ou orthopédiques obtenu en dissolvant du diméthylamino-phénéthanol dans du méthacrylate de méthyle et en mélangeant avec une poudre de polyméthacrylate de méthyle et du péroxyde de benzoyle; un inhibiteur de polymérisation est ajouté de manière à allonger la durée de polymérisation.

La présente invention a pour but de procurer un ciment pour fixation de prothèse d'os dont la réaction de polymérisation soit moins fortement exothermique, dont la température au cours de sa prise reste inférieure à 56°C, température de coagulation des protéines, et dont l'accélérateur de décomposition du peroxyde organique ne peut pas migrer dans l'organisme après le durcissement définitif du ciment, et n'exerce donc pas d'effet toxique sérieux même à long terme.

Ce but est atteint selon l'invention par le ciment tel qu'il est défini par la revendication 1 ci-après. En ce qui concerne des exemples de mise en oeuvre préférée de ce ciment, référence est faite aux revendications secondaires.

Il est décrit ci-après à titre d'exemple une formulation de ciment selon l'invention.

Sa phase solide a la composition suivante:

| | |
|---|---|
| Poudre de polyméthacrylate de méthyle | 24 grammes |
| Poudre de copolymère de méthacrylate de méthyle et de méthacrylate de glycidyle (oligomère de masse molaire moyenne en nombre 1000 à 5000 g/mole) | 12 g |
| Peroxyde de benzoyle | 0,3 g |
| | 36,3 g |

Sa phase liquide a la composition suivante:

| | |
|---|---|
| Méthacrylate de méthyle | 10,9 g |
| Acide méthacrylique | 1,03 g |
| Méthacrylate de méthylanilylglycidyle | 0,8 g |
| | 12,73 g |

Le rapport pondéral phase solide/phase liquide est voisin de 3.

La masse z d'acide méthacrylique est déterminée selon la relation générale suivante:

$$z = m \cdot \frac{\bar{f}}{\overline{Mn}} \cdot \frac{M'}{f'}$$

m étant la masse de copolymère (dans le cas actuel méthacrylate de méthyle-méthacrylate de glycidyle),
$\bar{f}$ la fonctionnalité moyenne de ce copolymère,
$\overline{Mn}$ la masse molaire moyenne en nombre de ce copolymère,
M' la masse molaire de la molécule réactive introduite dans la phase liquide (ici l'acide méthacrylique), soit 86 $g.mole^{-1}$,
f' la fonctionnalité de cette molécule (ici f' = 1).

Les masses de peroxyde de benzoyle et de méthacrylate de méthylanilylglycidyle sont ajustées de façon à obtenir le temps de prise en masse désiré, le temps de polymérisation étant d'environ 12 minutes.

On peut rajouter à la phase solide des charges telles qu'un radio-opacifiant (notamment le dioxyde de zirconium) et à la phase liquide d'autres monomères (tels les méthacrylates de butyle ou d'isobutyle) et des stabilisants.

La prise en masse du ciment s'effectue en deux étapes successives, correspondant à deux réactions différentes, après le mélange des phases liquide et solide. La viscosité du mélange avant sa prise en masse n'est pas plus élevée que celle d'un ciment connu, en dépit de la proportion élevée de phase solide.

La première étape est rapide et assure une prise en masse immédiate et le maintien de la prothèse; la seconde étape est plus lente et son rôle est de consolider le ciment.

**1°) Etape polymérisation radicalaire**

La prise en masse est tout d'abord assurée par la polymérisation radicalaire des monomères acryliques présents, le méthacrylate de méthyle, l'acide méthacrylique et le méthacrylate de méthylanilylglycidyle.

Les radicaux libres amorceurs de cette polymérisation sont créés par la réaction du méthacrylate de méthylanilylglycidyle sur le peroxyde de benzoyle. Ils amorcent alors lentement la polymérisation radicalaire du méthacrylate de méthyle et de l'acide méthacrylique, mais incorporent également dans les chaînes macromoléculaires en formation, les molécules de méthacrylate de méthylanilylglycidyle, qui comportent une double liaison. Celui-ci joue donc en même temps un rôle d'accélérateur de décomposition de l'amorceur et un rôle de monomère.

La propagation des chaînes de polymère est ensuite accélérée par la dissolution partielle de la poudre de polyméthacrylate de méthyle et de copolymère méthacrylate de méthyle-méthacrylate de glycidyle.

Cet effet d'accélération de la prise par augmentation de la viscosité (effet de gel) a déjà lieu dans les ciments connus. La polymérisation est de plus en plus rapide et, l'ouverture des doubles liaisons étant une réaction exothermique, il s'ensuit un dégagement de chaleur important.

Dans les ciments connus, une partie de la chaleur dégagée est absorbée par la poudre de polyméthacrylate de méthyle. Le nouveau ciment contient en plus de cette poudre une quantité assez importante de copolymère qui absorbe également une partie de la chaleur dégagée. La faible masse molaire des oligomères (méthacrylate de méthyle-méthacrylate de glycidyle) permet de réduire (sans modifier la viscosité) la proportion de méthacrylate de méthyle liquide et donc d'abaisser en proportion le dégagement thermique.

Des essais calorimétriques, réalisés dans les mêmes conditions, d'une part sur des mélanges contenant comme catalyseur de polymérisation de la diméthyl-para-toluidine a la place du méthacrylate de méthylanilylglycidyle, et ne contenant pas de copolymère de méthacrylate de méthyle et de méthacrylate de glycidyle, d'autre part sur des mélanges tels que celui décrit ci-dessus, montrent pour les premiers une température maximale de 80° - 90°C, pour les seconds une température maximale de 55°C. Celle-ci est inférieure à la température de coagulation des protéines (56°C), et risque donc beaucoup moins d'altérer le tissu osseux proche de la zone de mise en place du ciment.

Par ailleurs, le remplacement d'une amine tertiaire, relativement toxique, telle que la diméthyl-para-toluidine, par un composé jouant le même rôle d'accélérateur, mais se copolymérisant avec le monomère principal, réduit dans une très large mesure la toxicité à long terme due à cette molécule.

**2°) Formation d'un réseau tridimensionnel**

A la fin de la polymérisation radicalaire, le ciment est déjà dur, mais il contient des chaînes portant des fonctions réactives qui vont entraîner la formation d'un réseau tridimensionnel. La cinétique de formation d'un tel réseau est lente par rapport à celle de l'étape de polymérisation radicalaire; elle nécessite environ une semaine pour que la structure soit stabilisée.

Il existe alors dans le milieu

- d'une part des chaînes formées radicalairement dans la première étape, comportant quelques fonctions carboxyles réparties aléatoirement dans les chaînes, que l'on peut schématiser par:

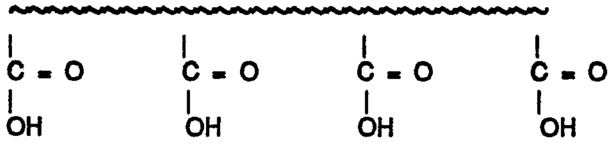

Elles contiennent essentiellement des motifs méthacrylate de méthyle et quelques motifs méthacrylate de méthylanilylglycidyle, ainsi que quelques motifs acide méthacrylique,

- d'autre part des chaînes oligomères de copolymère statistique de méthacrylate de méthyle et de glycidyle qui ont été introduites avec la phase solide. On peut les schématiser par:

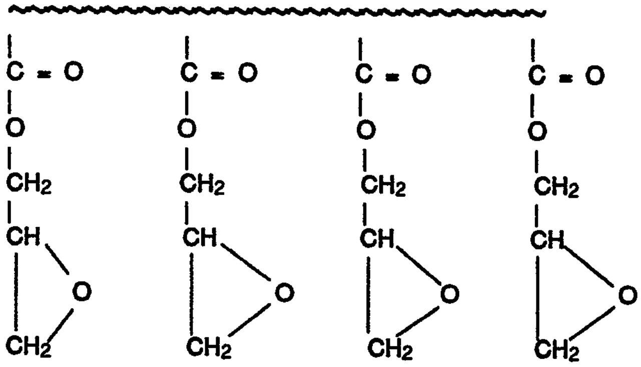

Les fonctions acide et les fonctions oxiranne réagissent au sein même du ciment avec ouverture du cycle oxiranne et formation d'hydroxyles. Ainsi deux chaînes indépendantes se relient par une liaison covalente. De tels réseaux se développent au sein du ciment et forment au bout d'environ une semaine une véritable trame qui consolide le ciment et empêche tout fluage. Le ciment comporte en fin de compte des réseaux semi-interpénétrés par des chaînes linéaires de polyméthacrylate de méthyle.

Le ciment ainsi formé présente des propriétés mécaniques aussi bonnes que celles des ciments connus, et il conserve la même facilité de malaxage que ceux-ci, bien que la proportion de phase solide y soit plus élevée.

Il ne présente par contre plus l'échauffement excessif au moment de la prise et la toxicité due à l'accélérateur de la décomposition du peroxyde organique que manifestaient ceux-ci.

Pour la mise en place du ciment de l'invention dans une prothèse, on mélange par exemple les phases solide et liquide pendant 2 à 3 minutes, on laisse reposer le mélange 5 à 6 minutes, on l'introduit dans l'os au bout de 7 à 8 minutes, soit à la main, soit à l'aide d'une seringue.

Bien que la composition du ciment qui a été décrite ci-dessus paraisse la formulation préférable peur la mise en oeuvre de l'invention, on peut remplacer certains de ses éléments par d'autres qui joueraient un rôle équivalent, ou modifier dans une certaine mesure les proportions, suivant la durée que l'on désire pour disposer d'une pâte encore malaxable, notamment en ce qui concerne les proportions de peroxyde et de méthacrylate de méthylanilylglycidyle. On peut utiliser d'autres couples oligomères/molécules réactives, par exemple:

| Oligomères introduits dans la phase solide | Molécules réactives introduites dans la phase liquide |
|---|---|
| Copolymères de méthacrylate de méthyle et d'acide méthacrylique | a) multiépoxyde ($\bar{f}' \geq 2$), par exemple le diglycidyl-éther du bisphénol A<br>ou<br>b) un méthacrylate ou acrylate comportant une fonction oxiranne (méthacrylate de glycidyle) |
| Copolymères de méthacrylate de méthyle et d'un acrylate ou méthacrylate comportant une fonction amine secondaire | comme ci-dessus |
| Copolymères de méthacrylate de méthyle et de méthacrylate | a) multiacide soluble dans la phase liquide ($\bar{f}' \geq 2$) de glycidyle<br>b) méthacrylate ou acrylate comportant une fonction acide carboxylique (acide méthacrylique ou acide acrylique) ou une fonction amine secondaire<br>c) multiamine primaire ou secondaire soluble dans la phase liquide et n'inhibant pas la première étape de polymérisation radicalaire. |

On peut faire varier la densité du réseau en modifiant le taux de méthacrylate de glycidyle dans le copolymère méthacrylate de méthyle-méthacrylate de glycidyle.

On peut modifier la flexibilité des chaînes de copolymère en y introduisant un troisième motif monomère qui abaisserait la température de transition vitreuse, par exemple un acrylate d'éthyle, de butyle ou d'éthyl-2-hexyle, un méthacrylate de n-butyle, d'isobutyle ou de glycidyle, l'acide acrylique, l'acide méthacrylique ou le diglycidyléther du bisphénol A.

## Revendications

1. Ciment pour fixation de prothèses d'os, notamment d'endoprothèses d'articulations, contenant:

a) une phase solide comprenant d'une part des poudres de polyméthacrylate de méthyle et d'un copolymère de méthacrylate de méthyle et de méthacrylate ou d'acrylate d'un autre alcool, et d'autre part un peroxyde organique générateur de radicaux libres, et
b) une phase liquide comprenant du méthacrylate de méthyle et un accélérateur de la décomposition du peroxyde organique, caractérisé en ce que, dans ladite phase solide, ledit copolymère est un oligomère, et ledit alcool est un alcool aliphatique porteur d'une fonction réactive susceptible de réagir ultérieurement avec une molécule de ladite phase liquide, et en ce que ladite phase liquide comprend, comme accélérateur de la décomposition dudit peroxyde organique, un méthacrylate ou acrylate porteur d'une fonction amine tertiaire permettant une prise en masse du ciment en quelques minutes, et comme agent de réticulation lente et non exothermique un composé comportant dans sa molécule au moins deux fonctions susceptibles de réagir avec ladite fonction réactive dudit alcool, ou bien un composé comportant dans sa molécule une double liaison et une fonction susceptible de réagir avec ladite fonction réactive dudit alcool, de façon à générer un polymère multifonctionnel agissant comme agent de réticulation.

2. Ciment selon la revendication 1, caractérisé par le fait que ledit oligomère est un oligomère de méthacrylate de méthyle et d'un méthacrylate portant une fonction oxiranne.

3. Ciment selon la revendication 2, caractérisé par le fait que ledit oligomère est un oligomère de méthacrylate de méthyle et de méthacrylate de glycidyle.

4. Ciment selon l'une des revendications précédentes, caractérisé par le fait que ledit accélérateur de la décomposition dudit peroxyde organique permettant une prise en masse du ciment en quelques minutes est le méthacrylate de méthylanilylglycidyle.

5. Ciment selon l'une des revendications précédentes, caractérisé par le fait qu'il contient environ trois parties de phase solide pour une partie de phase liquide.

## Patentansprüche

1. Zement zur Befestigung von Knochenprothesen, insbesondere von Gelenkendoprothesen, der als Bestandteile enthält:

a) eine feste Phase, die einerseits in Pulverform Polymethylmethacrylat und ein Kopolymer von Methylmethacrylat und ein Methacrylat oder Acrylat eines anderen Alkohols, und andererseits ein organisches Peroxid zur Erzeugung freier Radikale aufweist, und
b) eine flüssige Phase, die Methylmethacrylat und einen Beschleuniger zur schnelleren Zersetzung des organischen Peroxids aufweist, dadurch gekennzeichnet, daß in der festen Phase das Kopolymer ein Oligomer und der Alkohol ein aliphatischer Alkohol ist, der eine reaktive Funktion zur späteren Reaktion mit einem Molekül der flüssigen Phase aufweist, und daß die flüssige Phase als Beschleuniger für schnellere Zersetzung des organischen Peroxids ein Methacrylat oder Acrylat mit einer tertiären Aminfunktion, welches ein Festwerden des Zements in einigen Minuten erlaubt, und als Mittel zur langsamen und nicht exotherme Vernetzung eine Verbindung aufweist, die in ihrem Molekül mindestens zwei Funktionen enthält, die mit der reaktiven Funktion des Alkohols reagieren können oder eine Verbindung, die in ihrem Molekül eine Doppelbindung sowie eine Funktion besitzt, die mit der reaktiven Funktion des Alkohols reagieren kann, so daß ein multifunktionales Polymer als Vernetzungsmittel erzeugt wird.

2. Zement nach Anspruch 1, dadurch gekennzeichnet, daß das Oligomer ein Oligomer von Methylmethacrylat und von einem Methacrylat mit einer Oxiranfunktion ist.

3. Zement nach Anspruch 2, dadurch gekennzeichnet, daß das Oligomer ein Oligomer von Methylmethacrylat und von Glyzidylmethacrylat ist.

4. Zement nach einem der vorhergehenden Ansprüche, dadurch gekennzeichnet, daß der Beschleuniger zur schnelleren Zersetzung des organischen Peroxids und zum Verfestigen des Zements in einigen Minuten das Methylanilylglyzidyl-Methacrylat ist.

5. Zement nach einem der vorhergehenden Ansprüche, dadurch gekennzeichnet, daß er ungefähr drei Anteile fester Phase je Anteil flüssiger Phase enthält.

**Claims**

1. A cement for fixing a bone prosthesis, and in particular an endoprosthesis for a joint, the cement containing:

a) a solid phase comprising, on the one hand, polymethylmethacrylate and a copolymer of methyl methacrylate with an acrylate or a methacrylate of another alcohol in powder form, and on the other hand an organic peroxide suitable for generating free radicals, and
b) a liquid phase comprising methyl methacrylate and an accelerator for accelerating decomposition of the organic peroxide, the cement being characterized in that: in said solid phase, said copolymer is an oligomer, and said alcohol is an aliphatic alcohol carrying a reactive function suitable for subsequent reaction with a molecule in said liquid phase, and in that said liquid phase includes as the accelerator for accelerating decomposition of said organic peroxide an acrylate or a methacrylate carrying a tertiary amine function enabling the cement to set in bulk in a few minutes, and as a slow and non-exothermal cross-linking agent a compound having at least two functions in its molecule suitable for reacting with said reactive function of said alcohol, or else a compound including a double bond in its molecule and a function suitable for reacting with said reactive function of said alcohol, in such a manner as to generate a multifunctional polymer acting as a cross-linking agent.

2. A cement according to claim 1, characterized in that said oligomer is an oligomer of methyl methacrylate and of a methacrylate carrying an oxirane function.

3. A cement according to claim 2, characterized in that said oligomer is an oligomer of methyl methacrylate and of glycidyl methacrylate.

4. A cement according to any preceding claim, characterized in that said accelerator for accelerating decomposition of said organic peroxide enabling the cement to set in its bulk in a few minutes is methylanilylglycidyl methacrylate.

5. A cement according to any preceding claims, characterized in that it contains about three parts solid phase to one part liquid phase.